# EUROPEAN PATENT SPECIFICATION

(11) **EP 2 102 226 B1**
(45) Date of publication and mention of the grant of the patent: **22.08.2012**
(21) Application number: 06842454.8
(22) Date of filing: 12.12.2006
(51) Int. Cl.: C07H 17/00, A61K 31/7042, A61P 19/02

(54) **MACROLIDE COMPOUNDS ENDOWED WITH ANTIINFLAMMATORY ACTIVITY**
MAKROLIDVERBINDUNGEN MIT ENTZÜNDUNGSHEMMENDER WIRKUNG
COMPOSÉS MACROLIDES DOTÉS D'UNE ACTIVITÉ ANTI-INFLAMMATOIRE

(43) Date of publication of application: 23.09.2009
(73) Proprietor: ZAMBON S.p.A., 20091 Bresso MI (IT)
(72) Inventor: MEREU, Andrea, 22070 Grandate (IT); MORIGGI, Ermanno, 21052 Busto Arsizio (IT); NAPOLETANO, Mauro, 20127 Milano (IT); PELLACINI, Franco, 20151 Milano (IT)
(74) Representative: Colombo, Stefano Paolo
(86) International application number: PCT/IB2006/054776
(87) International publication number: WO 2008/072034

(56) References cited:
- WO-A-2005/075494
- WO-A-2007/054904
- WO-A-2007/060627
- WO-A2-2005/085266
- NAKAGAWA, S. ET AL.: "Inhibitory action of telithromycin against Shiga toxin and endotoxin" BIOCHEMICAL AND BIOPHYSICAL RESEARCH COMMUNICATIONS, ACADEMIC PRESS INC. ORLANDO, FL, US, vol. 310, no. 4, 31 October 2003 (2003-10-31), pages 1194-1199, XP004464090 ISSN: 0006-291X
- ARAUJO, F. G. ET AL.: "Inhibition of secretion of interleukin-1alpha and tumor necrosis factor alpha by the ketolide antibiotic telithromycin" ANTIMICROBIAL AGENTS AND CHEMOTHERAPY, AMERICAN SOCIETY FOR MICROBIOLOGY, WASHINGTON, DC, US, vol. 46, no. 10, October 2002 (2002-10), pages 3327-3330, XP002373935 ISSN: 0066-4804
- Grossman: "The Art of Writing Reasonable Organic Reaction Mechanisms", 1999, Springer, New York ISBN: 0-387-98540-9 pages v, vi-1,

## Description

The present invention relates to macrolides with antiinflammatory activity and, more particularly, relates to 3'-amide ketolides with antiinflammatory activity, to pharmaceutically acceptable salts thereof and to pharmaceutical compositions containing them as active ingredient. It is known that various antibiotics, in particular the class of erythromycin-based 14-atom macrolides, are endowed with antiinflammatory properties in addition to their antibacterial activity [Clin. Immunother., (1996), 6, 454-464].

Erythromycin is a natural macrolide (The Merck Index, 13th edition, No 3714, page 654) that has displayed very broad clinical use in the treatment of infections caused by Gram-positive bacteria, a few Gramnegative bacteria or micoplasms.

The interest of the scientific community has recently turned towards the immunomodulatory and antiinflammatory activities of erythromycin and derivatives [Journal of Antimicrobial Chemotherapy, (1998), 41, Suppl. B, 37-46].

Macrolides have been found to be effective in the treatment of inflammatory pathologies such as panbronchiolitis [Thorax, (1997), 52, 915-918], bronchial asthma [Chest, (1991), 99, 670-673] and cystic fibrosis [The Lancet, (1998), 351, 420].

The in vitro activity of macrolides was found to be particularly effective in modulating the metabolic functions of a number of immune system cells such as neutrophils [The Journal of Immunology, (1997), 159, 3395-4005] and T lymphocytes [Life Sciences, (1992), 51, PL 231-236] and in modulating inflammation mediators such as interleukin-8 (IL8) [Am. J. Respir. Crit. Care Med., (1997), 156, 266-271] or interleukin-5 (IL-5) [patent applications EP 0 775 489 and EP 0 771 564 in the name of Taisho Pharmaceutical Co., Ltd].

Neutrophils in particular are the first cell line recruited at the site of infection or of tissue lesion in the early stages of an inflammatory response.

A non-physiological accumulation of neutrophils in the inflamed tissue, their activation, the subsequent release of proteases and the increase in the production of reactive oxygen metabolites characterize a few forms of inflammatory response which, most often, degenerate into pathological conditions.

Thus, although neutrophils are essential in immune defence and in the inflammatory process, it is known that they are involved in pathologies derived from the majority of chronic inflammatory conditions and ischaemic reperfusion lesions (Inflammation and fever; Viera 'Stvrtinovà, Jan Jakubovsky and Ivan Hùlin; Academic Electronic Press, 1995).

The same paper discloses pathologies for which the influence of impaired functionality of the neutrophils during their genesis and/or development is confirmed: among these, mention is made of atherosclerosis, ischaemic reperfusion damage, rheumatoid arthritis, psoriasis, vasculitis and autoimmune-based glomerulonephritis, Crohn's disease and chronic pulmonary inflammations such as ARDS (adult respiratory distress syndrome).

COPD (chronic obstructive pulmonary disease) is a chronic pathology characterized by inflammation and gradual destruction of the pulmonary tissue caused by the massive presence of activated neutrophils with consequent release of metalloproteases and an increase in the production of oxygen radicals [Am. J. Respir. Crit. Care Med., 1996, 153, 530-534] [Chest, 2000, 117 (2 Suppl.), 10S-14S].

The administration of macrolides to asthmatic individuals is accompanied by a reduction in the hypersecretion and bronchial hypersensitivity consequent to their anti-oxidative and antiinflammatory interaction with phagocytes and in particular with neutrophils; this interaction is thought to impede many bioactive lipids, involved in the pathogenesis of bronchial asthma, from carrying out their proinflammatory membrane-destabilizing activity (Inflammation, Vol. 20, No. 6, 1996).

Treatment with erythromycin, at low doses for long periods, is described as being effective in reducing bronchial hypersensitivity in the case of asthma sufferers (Miyatake H. et al Chest, 1991, 99, 670-673, already cited).

In another study, it is demonstrated that the same treatment, in the case of COPD sufferers, can significantly reduce the frequency and the risk of exacerbation caused by acute respiratory infections (CHEST 2001, 120, 730-733).

The results obtained are not attributable to the antibiotic activity of macrolides, but to the inhibition of the expression and release of inflammatory cytokines.

This treatment, according to the abovementioned article, should preferably be restricted to patients at high risk of exacerbation of COPD on account of the potential risk of development of resistant pathogenic strains.

The particular therapeutic efficacy of macrolides in pathologies in which conventional antiinflammatory drugs, for instance corticosteroids, have proved to be ineffective [Thorax, (1997), 52, 915-918, already cited] justifies the appreciable interest with regard to this novel potential class of anti-inflammatories.

However, the fact that standard macrolides have potent antibacterial activity does not allow their broadened use in the chronic treatment of inflammatory processes not caused by pathogenic microorganisms; the reason for this is that this may give rise to the rapid development of resistant strains.

It would therefore be desirable to have available novel substances with a macrolide structure that show antiinflammatory activity and that are simultaneously free of antibiotic properties.

For greater clarity, the formula of erythromycin is given, in which is indicated the numbering adopted in the present patent application.

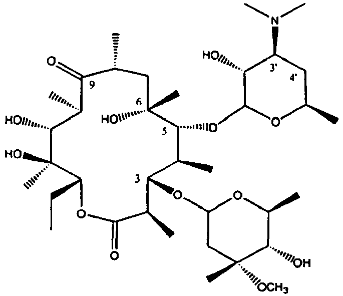

A number of classes of erythromycin derivatives with antibacterial activity are described in the literature.

In particular, telithromycin (The Merck Index, 13th edition, No 9199, page 1627) is a semisynthetic derivative of erythromycin A and is the first molecule belonging to a new family of broad-spectrum antibacterial agents closely related to macrolides, which are known as ketolides.

It has been demonstrated that telithromycin inhibits the production of inflammatory cytokines from Shiga toxin (Stx)-stimulated human peripheral blood monocytes [Biochem Biophys Res Commun 310, (2003), 1194-1199].

Moreover, thelithromycin has immunomodulatory activity as a result of the down-regulation of production of proinflammatory cytokines by human monocytes [Antimicrob Agents Chemother, (2002), 46:10, p. 3327-3330].

Telithromycin is used as a second-choice treatment in communityacquired infections sustained by penicillin-resistant and macrolide-resistant strains, in particular by group A beta-haemolytic streptococci and is also active against intracellular and atypical bacteria.

Patent application WO 99/16779 in the name of Abbott Laboratories discloses a number of classes of erythromycin-based ketolides modified in position 3' and 6-O-substituted, and to salts and esters thereof, with antibacterial activity.

The literature also discloses a number of classes of erythromycin derivatives with antiinflammatory activity.

For example, the abovementioned European patent applications in the name of Taisho claim erythromycin derivatives modified in positions 3, 9, 11 and 12, as potent inhibitors of IL-5 synthesis.

The use of erythromycin as an antiinflammatory agent that acts by reducing the release of interleukin-1 by means of inhibiting the mammalian glycoprotein mdr-P is claimed in patent application WO 92/16226 in the name of Smith-Kline Beecham Corporation.

Patent application WO 00/42055 in the name of the present applicant discloses 3'-desdimethylamino-9-oxime macrolides endowed with antiinflammatory activity and lacking antibiotic activity.

Patent application WO 04/013153 in the name of the present applicant discloses macrolide derivatives free of cladinose in position 3, which are endowed with antiinflammatory activity.

Patent application WO 04/039821 in the name of the present applicant discloses 9a-azalides free of cladinose in position 3, which are endowed with antiinflammatory activity.

Patent application WO2005/075494 in the name of the present applicant discloses erythromycin derivatives free of cladinose in position 3, which are endowed with anti-inflammatory activity.

Patent application WO2005/085266 discloses macrocyclic compounds useful as anti-infective, anti-proliferative, anti-inflammatory and prokinetic agents.

It has now been found, surprisingly, that ketolide derivatives structurally modified on the dimethylamino group in position 3' have no antibacterial activity and, at the same time, have pronounced antiinflammatory properties.

In particular, we have found, surprisingly, a novel class of telithromycin 3'-amide derivatives that are endowed with antiinflammatory activity and are substantially free of antibiotic properties.

One object of the present invention is thus compounds of formula 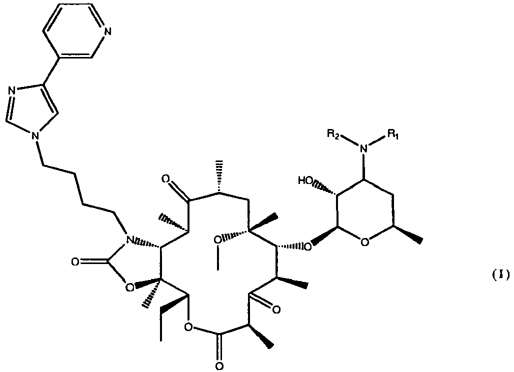 in which
R₁ is a group -X-R₃ in which
X is a group -C(=O)-, -C(=O)-NH- or -SO₂ and
R₃ is a hydrogen atom; a (C₁-C₁₀)alkyl group; a (C₁-C₄)alkoxy(C₁-C₄)alkyl group; a (C₅-C₇)cycloalkyl group; a phenyl, a heteroarylselected from pyrrole, thiophene, furan, imidazole, pyrazole, thiazole, isothiazole, isoxazole, oxazole, pyridine, pyrazine, pyrimidine, pyridazine, triazole and thiadiazole, a phenyl(C₁-C₄)alkyl group or a heteroaryl(C₁-C₄)alkyl group optionally substituted with 1 to 3 substituents chosen from a (C₁-C₄)alkyl group, a (C₁-C₄)alkoxy group and halogen; or a chain of formula

-(CH₂)ᵣ-Y-(CH₂)ₘ-A

in which
A is a phenyl or a heteroaryl selected from pyrrole, thiophene, furan, imidazole, pyrazole, thiazole, isothiazole, isoxazole, oxazole, pyridine, pyrazine, pyrimidine, pyridazine, triazole and thiadiazole, both optionally substituted with 1 to 3 substituents chosen from a (C₁-C₄)alkyl group, a (C₁-C₄)alkoxy group and halogen; or a group 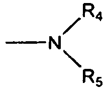 in which
R₄ and R₅, independently of each other, are a hydrogen atom, a (C₁-C₆)alkyl group, a benzyl, a (C₁-C₄)alkoxycarbonyl group or a benzyloxycarbonyl group;
Y represents O, S or NR₆ in which
R₆ is a hydrogen atom, a linear or branched (C₁-C₃)alkyl, a (C₁-C₃)alkoxycarbonyl group or a benzyloxycarbonyl group;
r is an integer between 1 and 3;
m is an integer between 0 and 3;
R₂ is a hydrogen atom, a (C₁-C₆)alkyl group, a benzyl group or has the meanings given for the substituent R₁,
and pharmaceutically acceptable salts thereof.

The compounds of formula I are antiinflammatory macrolides free of antibiotic activity and are therefore useful in the treatment and prophylaxis of inflammatory pathologies.

A further object of the present invention is the use of a compound of formula I as a medicament, in particular in the treatment of inflammatory pathologies.

The use of a compound of formula I in the preparation of a medicament for the treatment of gastrointestinal pathologies of inflammatory nature, for instance ulcerative colitis and Crohn's disease, is preferred.

Specific examples of (C₁-C₁₀)alkyl groups are methyl, ethyl, n-propyl, i-propyl, n-butyl, s-butyl, t-butyl, n-pentyl, 1-methylbutyl, 2-ethylpropyl, 3-methylbutyl, 3-methyl-2-butyl, n-hexyl, heptyl, octyl, nonyl, decyl and the like.

The term "(C₅-C₇)cycloalkyl group" means cyclopentyl, cyclohexyl and cycloheptyl.

The term "halogen" means a fluorine, chlorine, bromine or iodine atom.

The term "5- or 6-membered heteroaryl containing from 1 to 3 heteroatoms chosen from nitrogen, oxygen and sulfur" means heterocycles such as pyrrole, thiophene, furan, imidazole, pyrazole, thiazole, isothiazole, isoxazole, oxazole, pyridine, pyrazine, pyrimidine, pyridazine, triazole and thiadiazole.

It is clear to those skilled in the art that the substitution with partially or totally saturated forms of the heteroaryls and also the presence of other substituents on the aromatic rings (phenyl or heteroaryl) envisaged in the meanings of R₁ and R₂ gives rise to compounds that do not depart from the spirit of the invention.

Preferred compounds of formula I are those in which R₁ has the meaning given in formula I and R₂ is a hydrogen atom, a (C₁-C₆)alkyl group or a benzyl group.

Within this group, compounds in which R₂ is a (C₁C₃)alkyl group are even more preferred.

Belonging to this latter group, compounds that are also preferred are those in which R₁ is a group -X-R₃ in which X is a group -C(=O)-, -C(=O)-NH- or -SO₂- and R₃ is a hydrogen atom; a (C₁-C₆)alkyl group; a (C₁-C₄)alkoxy(C₁-C₄)alkyl group; a (C₅-C₇)cycloalkyl group; a phenyl, a five- or six-membered heteroaryl containing from one to three heteroatoms chosen from nitrogen, oxygen and sulfur, a phenyl(C₁-C₄)alkyl group or a heteroaryl(C₁-C₄)alkyl group optionally substituted with 1 to 3 substituents chosen from a (C₁-C₄)alkyl group, a (C₁-C₄)alkoxy group and halogen; or a chain of formula

-(CH₂)ᵣ-Y-(CH₂)ₘ-A

in which

A is a phenyl or a five- or six-membered heteroaryl containing from one to three heteroatoms chosen from nitrogen, oxygen and sulfur, both optionally substituted with 1 to 3 substituents chosen from a (C₁-C₄)alkyl group, a (C₁-C₄)alkoxy group and halogen; or a group 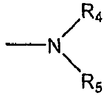 in which
R₄ and R₅, independently of each other, are a hydrogen atom, a (C₁-C₄)alkyl group or a benzyl;
Y represents O, S or NR₆ in which R₆ is a hydrogen atom or a (C₁-C₃)-alkyl group;
r is an integer between 1 and 3; and
m is an integer between 0 and 3.

Within the scope of this group, compounds that are even more preferred are those in which R₁ is a group -X-R₃ in which R₃ is a (C₁-C₆)alkyl group; a methoxy(C₁-C₃)alkyl group; a (C₅-C₇)cycloalkyl group; a phenyl, a heteroaryl chosen from furan, thiophene, oxazole and pyridine, a benzyl or heteroaryl(C₁-C₄)alkyl group optionally substituted with 1 to 3 substituents chosen from a (C₁-C₄)alkyl group, a methoxy group and halogen; or a chain of formula

-(CH₂)ᵣ-Y-(CH₂)ₘ-A

in which
A is a phenyl or a heteroaryl chosen from furan, thiophene, oxazole and pyridine, both optionally substituted with a substituent chosen from a (C₁-C₄)alkyl group, a methoxy group and halogen; or a group: 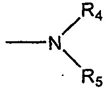 in which
R₄ and R₅, independently of each other, are a hydrogen atom or a (C₁-C₄)alkyl group;
Y represents O, S or NR₆ in which R₆ is a hydrogen atom;
r is an integer between 1 and 3; and
m is an integer between 0 and 2.

Within the scope of this latter group, compounds that are even more preferred are those in which R₁ is a group -X-R₃ in which X is a group -C(=O)- and R₃ is a (C₁-C₄)alkyl group or a phenyl.

Examples of pharmaceutically acceptable salts of the compounds of formula I are salts with organic or mineral acids such as hydrochloric acid, hydrobromic acid, hydriodic acid, nitric acid, sulfuric acid, phosphoric acid, acetic acid, tartaric acid, citric acid, benzoic acid, succinic acid and glutaric acid.

Another preferred class of compounds that are the object of the present invention is that in which R₂ is a methyl, R₁ is a group -X-R₃ in which X is a group -C(=O)-, -C(=O)-NH- or -SO₂- and R₃ is a hydrogen atom, a methoxymethyl group, a cyclohexyl group, a phenyl, a benzyl, a 4-methylphenyl, a 4-methoxyphenyl, a 4-fluorophenyl, a 2-furyl, a 3-pyridyl, a 2-thienyl, a 2-chloro-3-pyridyl, a 2-thienylmethyl, a 3-methyl-5-oxazolyl, a (4-methoxy-2-pyridylmethyl)oxymethyl group, a phenylthiomethyl group, an (N,N-dimethylaminoethyl)aminomethyl, methyl, ethyl, t-butyl or heptyl group.

The compounds:
3'-demethyl-3'-acetyltelithromycin; and
3'-demethyl-3'-benzoyltelithromycin,
are also particularly preferred.

The compounds of formula I that are the object of the present invention are prepared according to a synthetic scheme that involves demethylation of the dimethylamino group of telithromycin in position 3', followed by the amidation reaction of the primary or secondary amino group thus formed to give the compounds of formula I that are the object of the present invention.

The term "amidation reaction" means the operation for the introduction of the substituents X and R₃, defined in formula I in the meanings of R₁ and R₂, in one or more synthetic steps.

The indications to implement the abovementioned structural modifications to the ketolide derivatives are described more clearly hereinbelow.

Telithromycin is a known compound, which is commercially available and is prepared according to conventional techniques, for instance those described in patent EP 0 680 967 in the name of Hoechst Marion Roussel.

Starting with this substrate, the compounds of formula I are prepared via demethylation of the dimethylamino group in position 3' according to conventional techniques.

Thus, for example, telithromycin is treated with sodium acetate and iodine in the presence of an organic solvent as described in patent US 3 725 385 in the name of Abbott Laboratories.

Alternatively, the said substrate is reacted with a dialkyl azodicarboxylate in acetone as described in patent US 6 433 151 in the name of Aventis Pharma.

Functionalization of the primary or secondary amine group obtained in position 3' is performed via the use of amidation techniques known to those skilled in the art.

In particular, these synthetic techniques relate to the common preparations of amides, sulfonamides, ureas, sulfonylureas and urethanes from an amine substrate.

As indicated previously, the amidation reaction is completed by introduction of the substituents X and R₃ defined in formula I.

Generally, the substituents X and R₃ are introduced simultaneously onto the macrolide molecule.

Thus, for example, the preparation of amide or sulphonamide derivatives is generally performed by treating the 3'-demetliylated ketolide with suitable acyl chlorides or sulfonyl chlorides according to conventional techniques, for instance reaction of the abovementioned compounds in the presence of a base such as triethylamine and an organic solvent, for instance dichloromethane or tetrahydrofuran.

In addition, the preparation of the urea derivatives is preferably performed via the use of suitable isocyanates in the presence of an organic solvent, for instance dichloromethane.

Alternatively, the preparation of derivatives with structurally more complex amide chains is generally performed by synthesis according to stepwise processes.

Thus, for example, the 3'-demethylated derivative is treated with an omega-chloroalkanoic acid (acetic acid, propionic acid or butyric acid) and N-cyclohexylcarbodiimide in the presence of an organic solvent, for instance tetrahydrofuran, and the compound obtained is used as a substrate for the introduction of the end part of the amide chain, in particular of the compounds of formula I in which X is a group -C(=O)-and R₃ is a chain of formula -(CH₂)r-Y-(CH₂)m-A.

The procedural choices will be determined, as and when necessary, by technical requirements finally having the purpose of optimizing the synthetic process of the product under consideration.

As stated previously, the compounds of formula I that are the object of the present invention are endowed with antiinflammatory activity and are free of antibiotic activity.

The pharmacological activity of the compounds of formula I was evaluated in models of cutaneous inflammation compared with known macrolides such as erythromycin, telithromycin and azithromycin, which are endowed with both antiinflammatory activity and antibiotic activity.

The antiinflammatory activity was evaluated by means of inhibition of the oedema in mouse ear induced with PMA (phorbol myristate acetate).

In all the experiments, the compounds that are the object of the present invention were found to be very active as antiinflammatory agents and the antiinflammatory activity was found to be greater than that of the comparative compounds.

The antibiotic activity was evaluated "in vitro" as the capacity to inhibit the growth of bacterial strains sensitive to erythromycin and telithromycin.

In particular, the antibiotic activity of the compounds was evaluated by turbidimetric assay in which the measurement of the bacterial growth is given by the increase in the turbidity/absorbance recorded spectrophotometrically in the culture broth.

The antibiotics erythromycin and telithromycin were used simultaneously with the test compounds as reference standards for the bacterial growth-inhibiting activity.

All the test compounds were found to be inactive towards bacterial growth, whereas, for the reference antibiotics erythromycin and telithromycin, substantial inhibition of the bacterial development associated with the concentrations used was measured.

The compounds of the present invention show no antibiotic activity and may therefore be used in chronic treatments of inflammatory processes without giving rise to any undesired resistance phenomena.

It is thus obvious to a person skilled in the art that the compounds of formula I, which are endowed with antiinflammatory activity and are free of antibiotic activity, may be useful in both acute and chronic treatment and in the prophylaxis of inflammatory pathologies, in particular of pathologies related to impaired cell functionality of neutrophils, for instance rheumatoid arthritis, vasculitis, glomerulonephritis, psoriasis, atopic dermatitis, ulcerative colitis, Crohn's disease, ischaemic reperfusion damage, septic shock, atherosclerosis, ARDS, COPD and asthma.

The therapeutically effective amounts will depend on the age and the general physiological state of the patient, the route of administration and the pharmaceutical formulation used; the therapeutic doses will generally be between about 10 and 2000 mg/day and preferably between about 30 and 1500 mg/day.

The compounds of the present invention for use in the treatment and/or prophylaxis of the pathologies indicated above will preferably be used in a pharmaceutical form suitable for oral, rectal, sublingual, parenteral, topical, transdermal and inhaled administration.

A further object of the present invention is thus pharmaceutical formulations containing a therapeutically effective amount of a compound of formula I or a salt thereof mixed with a pharmaceutically acceptable carrier.

The pharmaceutical formulations that are the object of the present invention may be liquids suitable for oral and/or parenteral administration, for instance drops, syrups, solutions or injectable solutions that are ready for use or prepared by diluting a lyophilizate, but are preferably solid or semi-solid, for instance, tablets, capsules, granules, powders, pellets, ovules, suppositories, creams, pomades, gels and ointments; or solutions, suspensions, emulsions or other forms suitable for inhaled and transdermal administration.

Depending on the type of formulation, in addition to a therapeutically effective amount of one or more compounds of formula I, these compositions will contain suitable solid or liquid excipients or diluents for pharmaceutical use and optionally other additives normally used in the preparation of pharmaceutical formulations, for instance thickeners, aggregating agents, lubricants, disintegrants, flavourings and colourings.

The pharmaceutical formulations that are the object of the invention may be produced in accordance with conventional techniques.

The ¹H-NMR spectra were acquired in CDCl₃ or d₆-DMSO solutions, using a Varian Gemini 200 MHz spectrometer. The chemical shifts are reported in δ units using CHCl₃ or DMSO as internal standard.

The HPLC/MS analyses were performed using a Gilson machine containing a Gilson Xterra RP18 column (5 µm, 4.6 x 50 mm) and using as detector a UV diode array (220 nm), a Finnigan AQA mass spectrometer (electron spray, positive or negative ionization) and an ELSD detector.

Conditions used: flow rate: 1.2 ml/min; column temperature: 40°C; elution gradient A/B (eluent A: 0.5% formic acid in water; eluent B: 0.5% formic acid in acetonitrile): t = 0 min., A/B = 95:5, t = 8 min., A/B = 5:95.

The examples that follow are now given for the purpose of illustrating the present invention more clearly.

### Example 1

### Preparation of 3'-demethyl-3'-acetyltelithromycin (compound 1)

To a solution of 3'-demethyltelithromycin (200 mg, 0.25 mmol) and triethylamine (70 µl, 0.5 mmol) in CH₂Cl₂ (3 ml) was added dropwise a solution of acetyl chloride (19.6 mg, 0.25 mmol) in CH₂Cl₂ (1 ml), and the resulting mixture was stirred for 2 hours. The reaction mixture was diluted with ethyl acetate (50 ml) and the organic phase was washed with 2N NH₄Cl (3 × 30 ml), 10% K₂CO₃ (2 × 30 ml), dried over anhydrous Na₂SO₄ and filtered, and the solvent was evaporated off. Compound 1 (168 mg, 80% yield) was obtained as a white solid.
[M+1]⁺ 840.77;
HPLC-ELSD: Rt = 4.63; purity 99.0%;
¹H-NMR (CDCl₃): δ 8.94 (d, 1H, J=1.7, C²*H* pyridine); 8.43 (dd, 1H, J₁=4.9, J₂=1.5, C⁶*H* pyridine); 8.06 (dt, 1H, J₁=8.0, J₂=2.0, C⁴*H* pyridine); 7.52 (s, 1H, NC*H*N imidazole); 7.24-7.38 (m, 2H, C⁵*H* pyridine + C*H*C imidazole); 4.9 (m, 1H); 4.36 (d, 1H, J=7.5); 4.20 (d, 1 H, J=8.4); 4.00 (t, 2H, J=7.3, CH₂N_{imidazole}); 2.54 (m, 4H, C*H*₃N+CH); 1.24 (d, 3H, J=6.9); 0.98 (d, 3H, J=7.0); 0.81 (t, 3H, J=7.4; H₁₅).

### Example 2

### Preparation of 3'-demethyl-3'-benzoyltelithromycin (compound 2)

To a solution of 3'-demethyltelithromycin (200 mg, 0.25 mmol) and triethylamine (70 µl, 0.5 mmol) in CH₂Cl₂ (3 ml) was added dropwise a solution of benzoyl chloride (35.2 mg, 0.25 mmol) in CH₂Cl₂ (1 ml), and the resulting mixture was stirred for 2 hours. The reaction mixture was diluted with ethyl acetate (50 ml) and the organic phase was washed with 2N NH₄Cl (3 × 30 ml) and with 10% K₂CO₃ (2 × 30 ml), dried over anhydrous Na₂SO₄ and filtered, and the solvent was evaporated off. After purification by Biotage chromatography (12M cartridge column, eluent 100/5/0.5 CH₂Cl₂/MeOH/NH₃), compound 2 (205 mg, 91% yield) was obtained as a white solid.
[M+1]⁺ 902.79;
HPLC-ELSD: Rt = 5.38; purity 99.9%;
¹H-NMR (CDCl₃): δ 8.93 (d, 1H, J=1.9, C²*H* pyridine); 8.41 (dd, 1H, J₁=4.8, J₂=1.6, C⁶*H* pyridine); 8.05 (dt, 1H, J₁=8.1, J₂=2.0, C⁴*H* pyridine); 7.51 (s, 1 H, NC*H*N imidazole); 7.20-7.38 (m, 7H, C⁵*H* pyridine + C*H*C imidazole + phenyl); 4.9 (m, 1 H); 4.7 (m, 1 H); 3.97 (t, 2H, J=7.3, CH₂N_{imidazole}); 3.53 (s, 1H, H₁₁); 3.05 (t, 2H, J=7.5, CH₂); 2.86 (s, 3H, C*H*₃N); 2.59 (m, 4H, CH₂CH₂); 1.11 (d, 3H, J=6.9); 0.97 (d, 3H, J=6.9); 0.80 (t, 3H, J=7.5; H₁₅).

### Example 3

### in vivo pharmacological activity:

### A) Acute contact dermatitis

### • Animals

Groups of 5 CD1 mice (18-24 g) were used.

### • Administration of the compounds

All the macrolide derivatives were dissolved in Trans-phase Delivery System (TPDS), a carrier containing 10% benzyl alcohol, 40% acetone and 50% isopropanol.

15 microlitres of the compounds (500 µg/ear), dissolved in TPDS, were applied topically to the inner surface of an ear; 30 minutes later, 12 microlitres of a solution of tetradecanoylphorbol acetate (TPA) at a concentration of 0.01% dissolved in acetone were applied to the same area.

Six hours later, the animals were sacrificed by inhalation of CO₂.

### • Evaluation of the results

The degree of oedema was calculated by subtracting the weight of a specified section of the untreated ear from that of the contralateral treated ear. To determine the degree of remission of the oedema, the weight difference of the groups treated with TPA + macrolides was then compared with the groups treated with TPA alone.

The activity of the macrolides was measured by using the modified method of Zunic et al. (1998): MDL (Lysyl) GDP, a non-toxic muramyl dipeptide derivative inhibits cytokine production by activated macrophages and protects mice from phorbol ester- and oxazolone-induced inflammation (J. Invest. Dermatol., 111(1), 77-82).

The results obtained for the compounds of formula I are given in Table 1 below.

**Table 1**

| **Compound** | **Oedema (% inhibition)** |
|---|---|
| Erythromycin | 42 |
| Azithromycin | 40 |
| Telithromycin | 79.7 |
| 1 | 81.7 |
| 2 | 81.8 |

### Example 4

### In vitro pharmacological activity:

### A) Antibiotic activity:

The antibiotic activity of the compounds was evaluated by means of turbidimetric assay as described in the literature (Keller R., Pedroso M.Z. et al. Occurrence of virulence-associated properties in Enterobacter cloacae. Infection and Immunity, pp. 645-649, Feb. 1998), (Saiman L., Burns J.L. et al. Evaluation of reference dilution test methods for antimicrobial susceptibility testing of Pseudomonas aeruginosa strain isolated from patients with cystic fibrosis. Journal of Clinical Microbiology, pp. 2987-2991, Sept. 1999).

The turbidimetric determination of the bacterial growth is given by the increase in the transmittance/absorbance recorded spectrophotometrically in the test culture broth. The antibacterial activity was evaluated as being the concentration of compound capable of inhibiting the development of a culture broth of the bacterial strain Escherichia coli (ATCC 25922) incubated in 96-well plates. The antibiotics erythromycin and telithromycin were used simultaneously with test compounds as reference standards for the bacterial growth-inhibiting activity. The compounds and the reference antibiotics were tested at 8 concentrations obtained by means of 1:2 serial dilutions in the incubation plate, starting with a concentration of 100 µM.

### • Preparation of the compounds:

For each test compound, a 200 µM concentration was prepared (doubled relative to the incubation concentration), by diluting 10 µl of a 10 mM stock-solution in DMSO, in 0.5 ml of Tryptic Soy Broth (TSB).

200 µl of the prepared solutions were distributed in a 96-well plate and, by means of 1:2 serial dilutions with TSB, the successive concentrations were obtained. The resulting plate at the end of the dilutions contained, in a volume of 100 µl/well, one compound per column in 8 concentrations corresponding to 100, 50, 25, 12.5, 6.25, 3.13, 1.56 and 0.78 µM at incubation. For the positive controls (bacterial growth) and negative controls (blank/sterility control) in the wells with two columns, 100 µl/well of TSB without compounds were distributed.

### • Execution of the test

The incubation was initiated by dispensing 100 µl/well of the bacterial suspension, using a multi-channel pipette, into the columns containing test compounds, reference macrolides and positive control. 100 µl of TSB were dispensed in the sterility control/blank column.

The bacterial growth was measured by reading the absorbance at 570 nm using a 96-well plate reader. The readings were taken at the start of the incubation and every 30 minutes from 2 hours up to 5 hours of incubation.

### • Evaluation of the data

The absorbance values for each well, measured at the start of the incubation, were subtracted from the readings taken at the subsequent incubation times. From the values obtained, the inhibitions were calculated as a percentage of lack of bacterial growth relative to 100% growth measured in the uninhibited control wells at the respective incubation times. The values of the concentrations that inhibit the bacterial development by 20%, 50% and 80% (IC 20, 50, 80%) were calculated using at least 3 incubation times for which the increase in absorbance appeared to be the most linear among the concentrations (generally between 150 and 240 minutes of incubation). For each selected time, by using the paired series of concentrations and inhibitions, the inhibiting concentrations were calculated by means of a sigmoidal fit. The values of the inhibiting concentrations indicated are the mean of the values calculated for at least three selected incubation times. For the inactive compounds, an IC > 100 is indicated, meaning that at the maximum test concentration (100 µM), no inhibition could be calculated.

The results obtained for the reference antibiotic macrolides and for the compounds of formula I, expressed in nmol/ml of the concentrations (IC) that inhibit the growth of the bacterial strain Escherichia coli (ATCC 25922) by 20, 50 and 80%, are given in Table 2 below:

**Table 2**

| **Compound** | **IC 20%** | **IC 50%** | **IC 80%** |
|---|---|---|---|
| Erythromycin | 3.0 | 7.8 | 24.3 |
| Telithromycin | 2.7 | 5.7 | 14.9 |
| 1 | > 100 | > 100 | > 100 |
| 2 | > 100 | > 100 | > 100 |

The data given in Table 2 clearly indicate that the compounds of formula I, which are the object of the present invention, are substantially free of antibiotic activity.

## Claims

1. Compound of formula 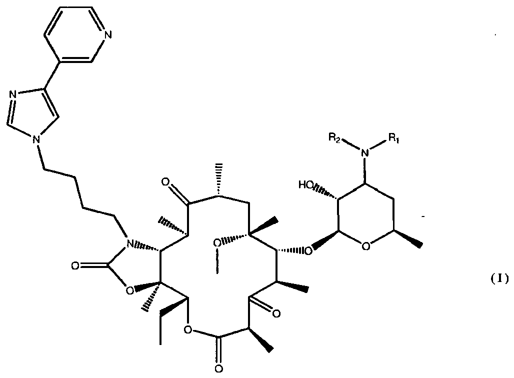 in which
R₁ is a group -X-R₃ in which
X is a group -C(=O)-, -C(=O)-NH- or -SO₂- and
R₃ is a hydrogen atom; a (C₁-C₁₀)alkyl group; a (C₁-C₄)alkoxy-(C₁-C₄)alkyl group; a (C₅-C₇)cycloalkyl group; a phenyl, a heteroaryl selected from pyrrole, thiophene, furan, imidazole, pyrazole, thiazole, isothiazole, isoxazole, oxazole, pyridine, pyrazine, pyrimidine, pyridazine, triazole and thiadiazole, a phenyl(C₁-C₄)alkyl group or a heteroaryl(C₁-C₄)alkyl group optionally substituted with 1 to 3 substituents chosen from a (C₁-C₄)alkyl group, a (C₁-C₄)alkoxy group and halogen; or a chain of formula
-(CH₂)ᵣ-Y-(CH₂)ₘ-A
in which
A is a phenyl or a heteroaryl selected from pyrrole, thiophene, furan, imidazole, pyrazole, thiazole, isothiazole, isoxazole, oxazole, pyridine, pyrazine, pyrimidine, pyridazine, triazole and thiadiazole, both optionally substituted with 1 to 3 substituents chosen from a (C₁-C₄)alkyl group, a (C₁-C₄)alkoxy group and halogen; or a group 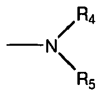 in which
R₄ and R₅, independently of each other, are a hydrogen atom, a (C₁-C₆)alkyl group, a benzyl, a (C₁-C₄)alkoxycarbonyl group or a benzyloxycarbonyl group;
Y represents O, S or NR₆ in which
R₆ is a hydrogen atom, a linear or branched (C₁-C₃)alkyl, a (C₁-C₃)alkoxycarbonyl group or a benzyloxycarbonyl group;
r is an integer between 1 and 3;
m is an integer between 0 and 3;
R₂ is a hydrogen atom, a (C₁-C₆)alkyl group, a benzyl group or has the meanings given for the substituent R₁,
and pharmaceutically acceptable salts thereof.

2. Compound according to Claim 1, in which R₂ is a hydrogen atom, a (C₁-C₆)alkyl group or a benzyl group.

3. Compound according to Claim 2, in which R₂ is a (C₁-C₃)alkyl group.

4. Compound according to Claim 3, in which R₁ is a group -X-R₃ in which X is a group -C(=O)-, -C(=O)-NH- or -SO₂ and R₃ is a hydrogen atom; a (C₁-C₆)alkyl group; a (C₁-C₄)alkoxy(C₁-C₄)alkyl group; a (C₅-C₇)cycloalkyl group; a phenyl, a heteroaryl selected from pyrrole, thiophene, furan, imidazole, pyrazole, thiazole, isothiazole, isoxazole, oxazole, pyridine, pyrazine, pyrimidine, pyridazine, triazole and thiadiazole, a phenyl(C₁-C₄)alkyl group or a heteroaryl(C₁-C₄)alkyl group optionally substituted with 1 to 3 substituents chosen from a (C₁-C₄)alkyl group, a (C₁-C₄)alkoxy group and halogen; or a chain of formula
-(CH₂)ᵣ-Y-(CH₂)ₘ-A
in which
A is a phenyl or a heteroaryl selected from pyrrole, thiophene, furan, imidazole, pyrazole, thiazole, isothiazole, isoxazole, oxazole, pyridine, pyrazine, pyrimidine, pyridazine, triazole and thiadiazole, both optionally substituted with 1 to 3 substituents chosen from a (C₁-C₄)alkyl group, a (C₁-C₄)alkoxy group and halogen; or a group 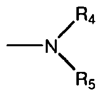 in which
R₄ and R₅, independently of each other, are a hydrogen atom, a (C₁-C₄)alkyl group or a benzyl;
Y represents O, S or NR₆ in which R₆ is a hydrogen atom or a (C₁-C₃)alkyl group;
r is an integer between 1 and 3; and
m is an integer between 0 and 3.

5. Compound according to Claim 4, in which R₁ is a group -X-R₃ in which R₃ is a (C₁-C₆)alkyl group; a methoxy(C₁-C₃)alkyl group; a (C₅-C₇)cycloalkyl group; a phenyl, a heteroaryl chosen from furan, thiophene, oxazole and pyridine, a benzyl or heteroaryl(C₁-C₄)alkyl group optionally substituted with 1 to 3 substituents chosen from a (C₁-C₄)alkyl group, a methoxy group and halogen; or a chain of formula
-(CH₂)ᵣ-Y-(CH₂)ₘ-A
in which
A is a phenyl or a heteroaryl chosen from furan, thiophene, oxazole and pyridine, both optionally substituted with a substituent chosen from a (C₁-C₄)alkyl group, a methoxy group and halogen; or a group: 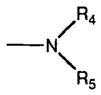 in which
R₄ and R₅, independently of each other, are a hydrogen atom or a (C₁-C₄)alkyl group;
Y represents O, S or NR₆ in which R₆ is a hydrogen atom;
r is an integer between 1 and 3; and
m is an integer between 0 and 2.

6. Compound according to Claim 5, in which R₁ is a group -X-R₃ in which X is a group -C(=O)- and R₃ is a (C₁-C₄)alkyl group or a phenyl.

7. Compound according to claim 1 in which R₂ is a methyl and R₁ is a group -X-R₃ in which X is a group -C(=O)- and R₃ is a methyl.

8. Compound according to claim 1 in which R₂ is a methyl and R₁ is a group -X-R₃ in which X is a group -C(=O)- and R₃ is a phenyl.

9. Process for preparing a compound of formula I according to claim 1, which comprises:
a. demethylation of the dimethyl amino group of telithromycin in position 3';
b. amidation reaction of the primary or secondary amine group thus formed as a product of the reaction in point a.

10. Pharmaceutical composition containing a therapeutically effective amount of a compound according to Claim 1 mixed with a pharmaceutically acceptable carrier.

11. Pharmaceutical composition according to Claim 10, which is useful for treating inflammatory pathologies.

12. Pharmaceutical composition according to Claim 11, which is useful for treating respiratory pathologies.

13. Pharmaceutical composition according to Claim 11, which is useful for treating gastrointestinal pathologies.

## Patentansprüche

1. Verbindung der Formel 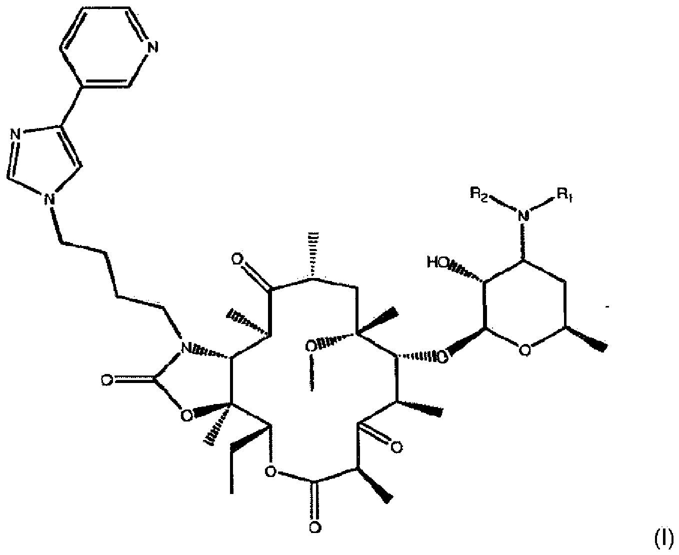 worin
R₁ für eine Gruppe -X-R₃ steht, worin
X für eine Gruppe -C(=O)-, -C(=O)-NH- oder -SO₂- steht und
R₃ für ein Wasserstoffatom; eine (C₁-C₁₀)Alkylgruppe; eine (C₁-C₄)Alkoxy-(C₁-C₄)alkylgruppe; eine (C₅-C₇)Cycloalkylgruppe; Phenyl, Heteroaryl, ausgewählt unter Pyrrol, Thiophen, Furan, Imidazol, Pyrazol, Thiazol, Isothiazol, Isoxazol, Oxazol, Pyridin, Pyrazin, Pyrimidin, Pyridazin, Triazol und Thiadiazol, eine Phenyl(C₁-C₄)alkylgruppe oder eine Heteroaryl(C₁-C₄)alkylgruppe, die gegebenenfalls mit 1 bis 3 unter einer (C₁-C₄)Alkylgruppe, einer (C₁-C₄)Alkoxygruppe und Halogen ausgewählten Substituenten substituiert sind; oder für eine Kette der Formel
-(CH₂)ᵣ-Y-(CH₂)ₘ-A
steht, worin
A für Phenyl oder Heteroaryl, ausgewählt unter Pyrrol, Thiophen, Furan, Imidazol, Pyrazol, Thiazol, Isothiazol, Isoxazol, Oxazol, Pyridin, Pyrazin, Pyrimidin, Pyridazin, Triazol und Thiadiazol, das jeweils gegebenenfalls mit 1 bis 3 unter einer (C₁-C₄)Alkylgruppe, einer (C₁-C₄)Alkoxygruppe und Halogen ausgewählten Substituenten substituiert ist; oder für eine Gruppe 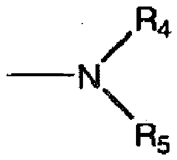 steht, worin
R₄ und R₅, unabhängig voneinander für ein Wasserstoffatom, eine (C₁-C₆)Alkylgruppe, Benzyl, eine (C₁-C₄)Alkoxycarbonyglruppe oder eine Benzyloxycarbonylgruppe stehen; Y für O, S oder NR₆ steht, worin
R₆ für ein Wasserstoffatom, eine lineare oder verzweigte (C₁-C₃)Alkyl-, eine (C₁-C₃)Alkoxycarbonylgruppe oder eine Benzyloxycarbonylgruppe steht;
r für eine ganze Zahl von 1 bis 3 steht;
m für eine ganze Zahl von 0 bis 3 steht;
R₂ für ein Wasserstoffatom, eine (C₁-C₆)Alkylgruppe, eine Benzylgruppe oder für einen Substituent mit der für R₁ angegebenen Bedeutung steht,
und pharmazeutisch akzeptable Salze davon.

2. Verbindung nach Anspruch 1, worin R₂ für ein Wasserstoffatom, eine (C₁-C₆)Alkylgruppe oder eine Benzylgruppe steht.

3. Verbindung nach Anspruch 2, worin R₂ für eine (C₁-C₃)Alkylgruppe steht.

4. Verbindung nach Anspruch 3, worin R₁ für eine Gruppe -X-R₃ steht, in der X für eine Gruppe -C(=O)-, -C(=O)-NH- oder -SO₂- steht und R₃ für ein Wasserstoffatom; eine (C₁-C₆)Alkylgruppe; eine (C₁-C₄)Alkoxy(C₁-C₄)alkylgruppe; eine (C₅-C₇)Cycloalkylgruppe; Phenyl, Heteroaryl, ausgewählt unter Pyrrol, Thiophen, Furan, lmidazol, Pyrazol, Thiazol, lsothiazol, lsoxazol, Oxazol, Pyridin, Pyrazin, Pyrimidin, Pyridazin, Triazol und Thiadiazol, eine Phenyl(C₁-C₄)alkylgruppe oder eine Heteroaryl(C₁-C₄)alkylgruppe, die gegebenenfalls mit 1 bis 3 unter einer (C₁-C₄)Alkylgruppe, einer (C₁-C₄)Alkoxygruppe und Halogen ausgewählten Substituenten substituiert sind; oder für eine Kette der Formel
-(CH₂)ᵣ-Y-(CH₂)ₘ-A
steht, worin
A für Phenyl oder Heteroaryl ausgewählt unter Pyrrol, Thiophen, Furan, Imidazol, Pyrazol, Thiazol, Isothiazol, lsoxazol, Oxazol, Pyridin, Pyrazin, Pyrimidin, Pyridazin, Triazol und Thiadiazol, das jeweils gegebenenfalls mit 1 bis 3 unter einer (C₁-C₄)Alkylgruppe, einer (C₁-C₄)Alkoxygruppe und Halogen ausgewählten Substituenten substituiert ist; oder für eine Gruppe 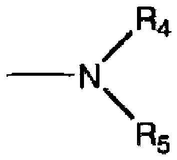 steht, worin
R₄ und R₅, unabhängig voneinander für ein Wasserstoffatom, eine (C₁-C₄)Alkylgruppe oder Benzyl stehen;
Y für O, S oder NR₆ steht, worin R₆ für ein Wasserstoffatom oder eine (C₁-C₃)Alkylgruppe steht;
r für eine ganze Zahl von 1 bis 3 steht; und
m für eine ganze Zahl von 0 bis 3 steht.

5. Verbindung nach Anspruch 4, worin R₁ für eine Gruppe -X-R₃ steht, in der R₃ für eine (C₁-C₆)Alkylgruppe; eine Methoxy(C₁-C₃)alkylgruppe; eine (C₅-C₇)Cycloalkylglruppe; Phenyl, Heteroaryl, ausgewählt unter Furan, Thiophen, Oxazol und Pyridin, Benzyl oder eine Heteroaryl(C₁-C₄)alkylgruppe, die gegebenenfalls mit 1 bis 3 unter einer (C₁-C₄)Alkylgruppe, einer Methoxygruppe und Halogen ausgewählten Substituenten substituiert sind; oder für eine Kette nach Formel
-(CH₂)ᵣ-Y-(CH₂)ₘ-A
steht, worin
A für Phenyl oder Heteroaryl, ausgewählt unter Furan, Thiophen, Oxazol und Pyridin, das jeweils gegebenenfalls durch einen unter einer (C₁-C₄)Alkylgruppe, einer Methoxygruppe und Halogen ausgewählten Substituenten substituiert ist; oder für eine Gruppe: 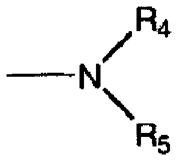 steht, worin
R₄ and R₅, unabhängig voneinander für ein Wasserstoffatom oder eine (C₁-C₄)Alkylgruppe stehen;
Y für O, S oder NR₆ steht, worin R₆ für ein Wasserstoffatom steht;
r für eine ganze Zahl von 1 bis 3 steht; und
m für eine ganze Zahl von 0 bis 2 steht.

6. Verbindung nach Anspruch 5, worin R₁ für eine Gruppe -X-R₃ steht, worin X für eine Gruppe -C(=O)- und R₃ für eine (C₁-C₄)Alkylgruppe oder Phenyl steht.

7. Verbindung nach Anspruch 1, worin R₂ für Methyl und R₁ für eine Gruppe -X-R₃ steht, worin X für eine Gruppe -C(=O)- und R₃ für Methyl steht.

8. Verbindung nach Anspruch 1, worin R₂ für Methyl und R₁ für eine Gruppe -X-R₃ steht, worin X für eine Gruppe -C(=O)- und R₃ für Phenyl steht.

9. Verfahren zur Herstellung einer Verbindung der Formel I nach Anspruch 1, bei dem man:
a. die Dimethylaminogruppe in Position 3' von Telithromycin demethyliert;
b. die als Produkt der Umsetzung in Schritt a. erhaltene primäre oder sekundäre Aminogruppe amidiert.

10. Pharmazeutische Zusammensetzung, enthaltend eine therapeutisch wirksame Menge einer Verbindung nach Anspruch 1, gemischt mit einem pharmazeutisch akzeptablen Träger.

11. Pharmazeutische Zusammensetzung nach Anspruch 10, geeignet zur Behandlung inflammatorischer Krankheiten.

12. Pharmazeutische Zusammensetzung nach Anspruch 11, geeignet zur Behandlung respiratorischer Krankheiten.

13. Pharmazeutische Zusammensetzung nach Anspruch 11, geeignet zur Behandlung gastronintestinaler Krankheiten.

## Revendications

1. Composé de formule 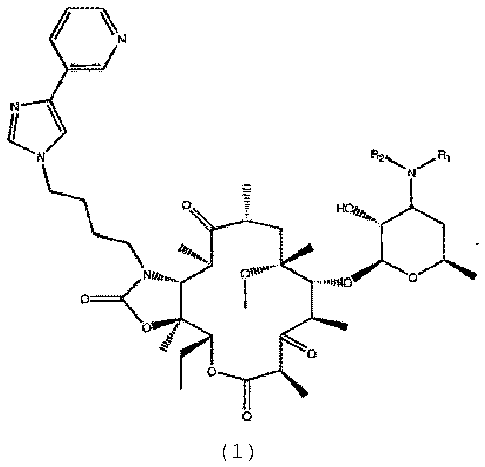 dans laquelle
R₁ est un groupe -X-R₃ dans lequel
X est un groupe -C(=O)-, -C(=O)-NH- ou -SO₂- et
R₃ est un atome d'hydrogène ; un groupe alkyle en C₁ à C₁₀ ; un groupe (alcoxy en C₁ à C₄)alkyle en C₁ à C₄ ; un groupe cycloalkyle en C₅ à C₇ ; un phényle, un hétéroaryle choisi parmi pyrrole, thiophène, furane, imidazole, pyrazole, thiazole, isothiazole, isoxazole, oxazole, pyridine, pyrazine, pyrimidine, pyridazine, triazole et thiadiazole, un groupe phényl(alkyle en C₁ à C₄) ou un groupe hétéroaryl(alkyle en C₁ à C₄) éventuellement substitué par 1 à 3 substituants choisis parmi un groupe alkyle en C₁ à C₄, un groupe alcoxy en C₁ à C₄ et un halogène ; ou une chaîne de formule
- (CH₂)ᵣ-Y-(CH₂)ₘ-A
dans laquelle
A est un phényle ou un hétéroaryle choisi parmi pyrrole, thiophène, furane, imidazole, pyrazole, thiazole, isothiazole, isoxazole, oxazole, pyridine, pyrazine, pyrimidine, pyridazine, triazole et thiadiazole, les deux éventuellement substitués par 1 à 3 substituants choisis parmi un groupe alkyle en C₁ à C₄, un groupe alcoxy en C₁ à C₄ et un halogène ; ou un groupe 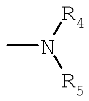 dans lequel
R₄ et R₅, indépendamment l'un de l'autre, sont un atome d'hydrogène, un groupe alkyle en C₁ à C₆, un benzyle, un groupe (alcoxy en C₁ à C₄)carbonyle ou un groupe benzyloxycarbonyle ;
Y représente 0, S ou NR₆, où
R₆ est un atome d'hydrogène, un alkyle en C₁ à C₃ linéaire ou ramifié, un groupe (alcoxy en C₁ à C₃)carbonyle ou un groupe benzyloxycarbonyle ;
r est un entier compris entre 1 et 3 ;
m est un entier compris entre 0 et 3 ;
R₂ est un atome d'hydrogène, un groupe alkyle en C₁ à C₆, un groupe benzyle, ou a les significations indiquées pour le substituant R₁,
et ses sels pharmaceutiquement acceptables.

2. Composé selon la revendication 1, dans lequel R₂ est un atome d'hydrogène, un groupe alkyle en C₁ à C₆ ou un groupe benzyle.

3. Composé selon la revendication 2, dans lequel R₂ est un groupe alkyle en C₁ à C₃.

4. Composé selon la revendication 3, dans lequel R₁ est un groupe -X-R₃ où X est un groupe -C(=O)-, -C(=O)-NH- ou -SO₂-et R₃ est un atome d'hydrogène ; un groupe alkyle en C₁ à C₆ ; un groupe (alcoxy en C₁ à C₄)alkyle en C₁ à C₄ ; un groupe cycloalkyle en C₅ à C₇ ; un phényle, un hétéroaryle choisi parmi pyrrole, thiophène, furane, imidazole, pyrazole, thiazole, isothiazole, isoxazole, oxazole, pyridine, pyrazine, pyrimidine, pyridazine, triazole et thiadiazole, un groupe phényl(alkyle en C₁ à C₄) ou un groupe hétéroaryl(alkyle en C₁ à C₄) éventuellement substitué par 1 à 3 substituants choisis parmi un groupe alkyle en C₁ à C₄, un groupe alcoxy en C₁ à C₄ et un halogène ; ou une chaîne de formule
- (CH₂)ᵣ-Y-(CH₂)ₘ-A
dans laquelle
A est un phényle ou un hétéroaryle choisi parmi pyrrole, thiophène, furane, imidazole, pyrazole, thiazole, isothiazole, isoxazole, oxazole, pyridine, pyrazine, pyrimidine, pyridazine, triazole et thiadiazole, les deux éventuellement substitués par 1 à 3 substituants choisis parmi un groupe alkyle en C₁ à C₄, un groupe alcoxy en C₁ à C₄ et un halogène ; ou un groupe 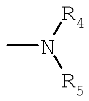 dans lequel
R₄ et R₅, indépendamment l'un de l'autre, sont un atome d'hydrogène, un groupe alkyle en C₁ à C₄ ou un benzyle ;
Y représente 0, S ou NR₆, où R₆ est un atome d'hydrogène ou un groupe alkyle en C₁ à C₃ ;
r est un entier compris entre 1 et 3 ; et
m est un entier compris entre 0 et 3.

5. Composé selon la revendication 4, dans lequel R₁ est un groupe -X-R₃ où R₃ est un groupe alkyle en C₁ à C₆ ; un groupe méthoxy(alkyle en C₁ à C₃) ; un groupe cycloalkyle en C₅ à C₇ ; un phényle, un hétéroaryle choisi parmi furane, thiophène, oxazole et pyridine, un groupe benzyle ou hétéroaryl(alkyle en C₁ à C₄) éventuellement substitué par 1 à 3 substituants choisis parmi un groupe alkyle en C₁ à C₄, un groupe méthoxy et un halogène ; ou une chaîne de formule
- (CH₂)ᵣ-Y-(CH₂)ₘ-A
dans laquelle
A est un phényle ou un hétéroaryle choisi parmi furane, thiophène, oxazole et pyridine, les deux éventuellement substitués par un substituant choisi parmi un groupe alkyle en C₁ à C₄, un groupe méthoxy et un halogène ; ou un groupe 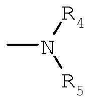 dans lequel
R₄ et R₅, indépendamment l'un de l'autre, sont un atome d'hydrogène ou un groupe alkyle en C₁ à C₄ ;
Y représente 0, S ou NR₆, où R₆ est un atome d'hydrogène ;
r est un entier compris entre 1 et 3 ; et
m est un entier compris entre 0 et 2.

6. Composé selon la revendication 5, dans lequel R₁ est un groupe -X-R₃ où X est un groupe -C(=O)- et R₃ est un groupe alkyle en C₁ à C₄ ou un phényle.

7. Composé selon la revendication 1, dans lequel R₂ est un méthyle et R₁ est un groupe -X-R₃ où X est un groupe -C(=O)-et R₃ est un méthyle.

8. Composé selon la revendication 1, dans lequel R₂ est un méthyle et R₁ est un groupe -X-R₃ où X est un groupe -C(=O)-et R₃ est un phényle.

9. Procédé pour préparer un composé de formule I selon la revendication 1, qui comprend :
a. la déméthylation du groupe diméthylamino de télithromycine en position 3' ;
b. une réaction d'amidation du groupe amine primaire ou secondaire ainsi formé en tant que produit de la réaction dans le point a.

10. Composition pharmaceutique contenant une quantité efficace, du point de vue thérapeutique, d'un composé selon la revendication 1 en mélange avec un véhicule pharmaceutiquement acceptable.

11. Composition pharmaceutique selon la revendication 10, qui est utile pour le traitement de pathologies inflammatoires.

12. Composition pharmaceutique selon la revendication 11, qui est utile pour le traitement de pathologies respiratoires.

13. Composition pharmaceutique selon la revendication 11, qui est utile pour le traitement de pathologies gastrointestinales.
